Europäisches Patentamt

European Patent Office

Office européen des brevets

(11) Publication number: **0 096 917**
**A1**

# (12) EUROPEAN PATENT APPLICATION

(21) Application number: **83200771.0**

(22) Date of filing: **31.05.83**

(51) Int. Cl.³: **C 07 H 15/04**

---

(30) Priority: **14.06.82 US 388379**

(43) Date of publication of application: **28.12.83**
**Bulletin 83/52**

(84) Designated Contracting States: **AT BE CH DE FR GB IT LI NL SE**

(71) Applicant: **THE PROCTER & GAMBLE COMPANY, 301 East Sixth Street, Cincinnati Ohio 45202 (US)**

(72) Inventor: **Farris, Diana Dunn, 3528 Danbury Road, Fairfield Ohio 45014 (US)**

(74) Representative: **Ernst, Hubert et al, PROCTER & GAMBLE EUROPEAN TECHNICAL CENTER Temselaan 100, B-1820 Strombeek-Bever (BE)**

---

(54) **Process for preparation of alkyl glycosides.**

(57) Preparatin of alkyl glycosides by acid catalyzed reaction of $C_8$ to $C_{25}$ alcohols with saccharides wherein the saccharide is incrementally or continuously added to the alcohol.

EP 0 096 917 A1

# PROCESS FOR PREPARATION OF ALKYL GLYCOSIDES

Diane D. Farris

## FIELD OF THE INVENTION

The invention pertains to the preparation of long chain alkyl glycosides by the direct reaction of a higher ($C_8$ to $C_{25}$) alcohol with a saccharide in the presence of an acid catalyst.

## BACKGROUND ART

U. S. Pat. No. 3,839,318, Mansfield, issued October 1, 1974, discloses a process for preparing alkyl glycosides by the direct, acid-catalyzed reaction of a higher alcohol and a saccharide, wherein the reaction mixture is kept in a single phase by choosing an appropriate alcohol:saccharide ratio between 6:1 and 1:1 (depending on the chain length of alcohol) and wherein the water of reaction is removed as soon as it is formed. The reaction produces a mixture of alkyl monoglycosides, and alkyl polyglycosides. As used herein, the term "alkyl monoglycoside" means an alkyl ether of a monosaccharide; the term "alkyl polyglycoside" means an alkyl ether of a polysaccharide and the term "alkyl glycoside" encompasses both alkyl monoglycosides and polyglycosides. The prefix "poly" herein means a number of repeating groups greater than 1, and is inclusive of the sometimes-used prefix "oligo."

For alkyl glycosides intended for detergent use (e.g., in formulating laundry products, hand dishwashing products and shampoos) it has been found desirable that certain compositional limits be met.

Specifically, the average chain length of the glycoside portion of the alkylglycoside should be from about 1.5 to about 3 (preferably from about 1.6 to about 2.75) units, the level of mono- and polysaccharide should be less than about 10%, the amount of alkyl polyglycoside in which the glycoside chain is 6 units or greater should be less than about 10% (preferably less than 5%), the alkyl monoglycoside content should be from about 20% to about 70% (preferably from about 30% to about 60%, most preferably from about 30% to about 50%) and the content of unreacted fatty alcohol should be less than 2% (preferably less than 0.5%). The alkyl chain length should be from about 8 to 25, preferably from about 12 to 18, and most preferably about 12 to 14 carbon atoms. It has been found that the process of U.S. Pat. No. 3,839,318 does not consistently produce alkyl glycosides which meet these compositional limits. In particular, the process of U.S. Pat. No. 3,839,318 produces a reaction product having higher than desired amounts of polysaccharides and of alkyl higher polyglycosides, insofar as optimum detergent use is concerned.

It is the object of the present invention to provide a process for the direct reaction of a higher alcohol and a saccharide to form high yields of alkyl glycosides which are especially useful in detergent applications.

All proportions and percentages expressed in this specification are "by weight" unless indicated otherwise.

## SUMMARY OF THE INVENTION

The present invention is directed to an improved process for preparing alkyl glycosides which are especially suitable for detergent utility. The improvement is achieved by incremental or continuous addition of saccharide to a mixture of higher alcohol and acid catalyst and simultaneously removing water of reaction as the reaction proceeds. The addition of saccharide is done at a rate which is sufficiently low to keep the reaction mix substantially in a single phase. The single reaction phase is indicative of low content of polysaccharides and alkyl higher glycosides, which are poorly soluble in the reaction mix.

## DETAILED DESCRIPTION OF THE PREFERRED EMBODIMENTS

The present invention is a process for preparing alkyl glycosides, comprising reacting a monosaccharide with at least a 50% molar excess of a $C_8$ to $C_{25}$ monohydric alcohol in the presence of an acid catalyst at a temperature of from about 80°C to about 150°C and continuously removing the water of reaction from the system, wherein the monosaccharide is continuously or incrementally added, to the alcohol and catalyst at a rate such that the amount of unreacted monosaccharide in the reaction mix is no more than about 10% by weight of said mix at any time and the average amount of unreacted monosaccharide in the said mix during the course of the reaction does not exceed about 5%. When the monosaccharide is added at ratios within these concentration parameters the reaction mix will be maintained in a substantially single phase condition. The 50% molar excess of alcohol over monosaccharide refers to the total amount of these reactants introduced into the reaction.

Conduct of the reaction in accordance with the present invention provides a direct, inexpensive and convenient method for producing high yields of alkyl glycosides having desirable detergent properties.

The monohydric alcohols containing from 8 to 25 carbon atoms used in the present invention may be primary or secondary alcohols, straight or branched chain, saturated or unsaturated, alkyl or aralkyl alcohols, ether alcohols, cyclic alcohols, or heterocyclic alcohols. In general, these alcohols are insoluble in water and have minimal solvent power for the saccharide molecule. Illustrative examples of the monohydric alcohols which may be employed in the present invention are octyl alcohol, nonyl alcohol, decyl alcohol, dodecyl alcohol, tridecyl alcohol, tetradecyl alcohol, pentadecyl alcohol, hexadecyl alcohol, heptadecyl alcohol, octadecyl alcohol, eicosyl alcohol, pentacosyl alcohol, oleyl alcohol, isoborneol alcohol, hydroabietyl alcohol, phenoxyethanol, phenoxypolyethoxyethanol containing five ethoxy groups, 2-methyl, 7-ethyl, 4-undecanol, and the like. A preferred group of alcohols are those having the formula ROH wherein R represents alkyl groups having from 8 to 25 carbon atoms. A particularly preferred group of alcohols are those wherein R represents an alkyl radical having from 12 to 18, preferably 12 to 14, carbon atoms. Preferably the alkyl is straight chain.

The saccharides used as reactants in the process herein are monosaccharides which can be alkylated in the "1" position, also sometimes called "reducing monosaccharides." These include hexoses and pentoses. Typical examples of suitable monosaccharides include glucose, manose, galactose, talose, allose, altrose, idose, arabinose, xylose, ribose, lyxose, and the like. For reasons of convenience, availability and low cost, the preferred starting product is glucose.

To facilitate reaction in the process herein it is preferred that the monosaccharide be finely ground to a particle size of less than about 300 microns or homogenized as a slurry in a portion of the alcohol which is used in the reaction. This slurry can then be fed to the remainder of alcohol which is in the reaction vessel and contains the acid catalyst.

The amount of alcohol and monosaccharide employed in the process will generally be such that the total molar ratio of alcohol to monosaccharide is from about 1.5:1 to about 6:1. Preferably a molar ratio of alcohol: monosaccharide of from about 2.5:1 to about 5:1 is employed. The most preferred ratio is from about 3:1 to about 4:1.

The acid catalyst used in the process herein may be any of those generally recognized in the art as being suitable for use in catalysis of the reaction between an alcohol and saccharide. Specific examples are sulfuric acid, hydrochloric acid, phosphoric acid, phosphorous acid, p-toluene sulfonic acid, boron trifluoride and sulfonic acid ion exchange resins. Sulfuric acid and p-toluene sulfonic acid are preferred catalysts.

The process herein is conducted at a temperature in the range of from about 80°C to about 150°C (preferably from about 100°C to about 140°C, and most preferably about 120°C to about 130°C). Water of reaction is removed as the reaction proceeds. If water of reaction is to be removed by distillation, the reaction will preferably be conducted at a reduced pressure of from about 0.1 mm to about 300 mm (preferably from about 0.1 mm to about 50 mm, and most preferably from about 3 to about 10 mm) of mercury, in a reaction vessel equipped with a distillation head. The distillation head is used to distill water of reaction from the vessel as the reaction proceeds. Instead of a distillation head, the reaction vessel can be equipped with other known means of removing water from a reaction mix. For example, a Dean-Stark trap can be used, in which case the reaction will be carried out at atmospheric pressure in the presence of a hydrocarbon solvent such as heptane or toluene to azeotrope the water of reaction during reflux. The amount of hydrocarbon to be employed is selected such that reflux will occur at the desired reaction temperature.

A mixture of the alcohol and acid catalyst is first formed in the reaction vessel. This can be done with all of the alcohol to be employed in the reaction, or up to 70% of the alcohol can be withheld to be used to form a slurry of the monosaccharide which is to be added to the reaction vessel. The amount of catalyst employed can vary over a wide range but is preferably at a weight amount of from about 0.03% to about 10% of the amount of monosaccharide.

In carrying out the process, the monosaccharide is added to the continuously stirred mixture of alcohol and catalyst either incrementally or continuously at a rate such that the amount of unreacted monosaccharide in the reaction mix is no more than about 10% (preferably no more than about 5%) of said mix at any time, and the average amount of unreacted monosaccharide in the said mix during the course of the reaction is no more than about 5% (preferably no more than about 3%) by weight of said mix. Addition of the monosaccharide at a rate which is sufficiently low to adhere to these concentration parameters will keep the reaction mix in a substantially single phase (i.e., visually clear) condition. As indicated above, the monosaccharide can be slurried in part of the alcohol to facilitate the feed of monosaccharide to the reaction vessel. The term "substantially single phase" is used herein to take into account the fact that with incremental addition of monosaccharide, when the monosaccharide is added to the reaction mixture there will be a short period of turbidity until the monosaccharide is dissolved into the single phase reaction mixture as a result of chemical reaction and/or solvation. Any permanent turbidity of the reaction mix is the result of the formation of excessive amounts of polysaccharides, alkyl higher polyglycosides, etc., indicating that monosaccharide has been added to the system at too fast a rate. On the other hand, when using continuous addition of monosaccharide there will be a slight turbidity in the reaction mix throughout the continuous period of addition. This is due to the small amount of unreacted monosaccharide which is always present. If addition is stopped, the turbidity will quickly disappear because this unreacted monosaccharide will quickly be used up in the reaction. So long as the initial low level of turbidity does not increase during the course of continuous addition of monosaccharide, undesired polymeric byproducts are not being formed, and the reaction mix is defined as "substantially clear" within the meaning of the present invention.

The concentration of unreacted monosaccharide in the reaction mix at any given time can be determined by gas chromatography analysis. A suitable technique is to determine the amount of monosaccharide (e.g., glucose) as the trimethylsilyl derivative by capillary column, high temperature gas chromatography using 1-nonanol as an internal standard. Samples are derivatized with a solution of trimethylsilylimidazole in pyridine. A relative response factor for glucose is determined from the analysis of a primary standard containing a known amount of pure alpha-D-glucose and a known amount of 1-nonanol. The response factor is calculated from the following equation:

$$RF \text{ (glucose)} = \left(\frac{Wt. \text{ glucose}}{Wt. \text{ 1-nonanol}}\right)\left(\frac{Area \text{ 1-nonanol}}{Area \text{ glucose}}\right).$$

Alpha- and Beta-D-glucose are assumed to have the same response factors. Crude reaction mixtures are then analyzed using a known amount of the same internal standard and the glucose concentration is calculated from the following equation:

$$\text{Wt.\% Glucose} = \left(\frac{\text{Wt. 1-nonanol}}{\text{Area 1-nonanol}}\right) \left(\frac{\text{Area glucose}}{\text{Wt. sample}}\right) (RF) \cdot 100$$

The analysis can be performed on any capillary gas chromatograph equipped with a splitter, flame ionization detector and integrator (HP 5880 or equivalent). The capillary column is a 15 meter, 0.25 mm I.D. DBl-15N fused silica or equivalent. The gas chromatograph is operated under the following conditions: injection port temperature, 360°C; detector temperature, 360°C; carrier gas, helium; flow rate, 1.0 ml/minute; initial column temperature, 100°C; temperature program rate, 10°C/minute; final column temperature, 350°C; final temperature hold time, 20 minutes; amount injected, 1.0 microliter.

If it is inconvenient to monitor the reaction analytically the rate of addition of monosaccharide can be established visually, i.e., by adding monosaccharide at a rate which maintains substantial visual clarity in the reaction mix. The actual rate of addition established to maintain substantial clarity will be dependent upon the many factors which can affect the rate of reaction such as catalyst concentration, reaction temperature, efficiency of water removal, reactivity of the particular alcohol and saccharide, mixing efficiency, etc. The appropriate rate of addition can be determined empirically by the visual method for any particular set of circumstances.

When the reaction is complete the catalyst in the reaction mixture is neutralized with base, and the product, if desired, is further purified by removal of some or all of the unreacted alcohol. As indicated previously herein, for detergent use the amount of alcohol in the reaction product should be reduced to 2% or less. The alcohol can be removed by solvent extraction or distillation. (See U.S. Pat. No. 3,839,318, incorporated herein by reference). If distillation is used, it is preferred that a thin film evaporator be used so the desired reaction product will be subjected to high temperatures for as brief a time as possible. (See European Patent Application 83200573.0, Mao et al., filed April 20, 1983, incorporated herein by reference).

The invention will be illustrated by the following examples.


### EXAMPLE I

A 1000 ml three-neck flask is equipped with a heating mantle and Thermo-Watch, a condenser and receiver, a mechanical stirrer, a vacuum pump and gauge and a liquid inlet. This is the reaction vessel. Separately, a 1000 ml beaker is fitted with a mechanical stirrer, dip tube, positive displacement pump with variable delivery rates and delivery lines connecting the dip tube to the liquid inlet of the reaction vessel.

380 grams (2.04 moles) of n-dodecanol are added to the reaction flask and heated to 125°C. This temperature is maintained throughout the course of the reaction. One gram of p-toluene sulfonic acid catalyst is added and the vessel pressure reduced to 5-10 mm Hg. A mixture of 120 grams (0.67 moles) glucose and 120 grams (0.64 moles) n-dodecanol is homogenized with a Gifford Wood high shear mixer to form a stable suspension and is added to the 1000 ml beaker. The slurry is metered from the beaker to the reaction vessel at a rate of 2.67 g/min. for 90 minutes. The reaction mix had a slight turbidity which did not increase during the course of the addition of the glucose slurry. The acid catalyst is neutralized with a saturated $Na_2CO_3$ solution.

The neutralized product is analyzed by gas chromatography and is found to contain the following: 65% unreacted alcohol, 2.8% glucose, 0.1% polysaccharide, 18.3% alkyl monoglycoside, 7.1% alkyldiglycoside, 4.0 alkyltriglycoside and 2.0 alkyl tetraglycoside. If this product is distilled in a Pope Wiped Film Evaporator operated at about 160-170°C and about 0.5 mm pressure to reduce the unreacted alcohol content to 0.5%, a product containing 8.1% glucose, 0.3% polysaccharide, 53.1% alkyl monoglycoside, 20.6% alkyl diglycoside, 11.6% alkyl triglycoside and 5.8% alkyl tetraglycoside and 0.5% unreacted alcohol will be obtained.

## EXAMPLE II

A 1000 ml three-neck flask is fitted with a heating mantle and Thermo-Watch, a condenser and receiver, a mechanical stirrer, a vacuum pump and gauge and a powder inlet.

500 grams of n-dodecanol (2.68 moles) are added to the reaction flask and heated to 125°C. This temperature is maintained throughout the course of the reaction. One gram of p-toluene sulfonic acid catalyst is added, followed by 20 grams of anhydrous glucose. The reaction vessel pressure is then reduced to 5-10 mm Hg. This is time 0 for purpose of timing reaction. At fifteen-minute intervals the vessel is allowed to come to atmospheric pressure and 20 grams of glucose are manually added. The flask is reevacuated. Incremental glucose addition is continued until 120 grams (0.67 mole) total (six increments of 20 grams each) have been added. The reaction mix is held at 125°C and 5-10 mm Hg. for fifteen minutes after the last addition of glucose, then brought to atmospheric pressure and then the acid catalyst is neutralized with a stoichiometric amount of saturated $Na_2CO_3$. At each addition of glucose, the reaction mix becomes cloudy, but then becomes substantially clear after about 5-10 minutes of continued stirring.

Percent unreacted glucose in the reaction mix is determined immediately after the first addition of glucose at time 0, before and after glucose addition at 15, 45 and 60 minutes, and at the end of the reaction (90 minute time point). Results are obtained by gas chromatography except for the results reported after addition at 15 and 60 minutes, which are calculated. Results are shown in the following table.

TABLE I

| Time (Min.) | % by Wt. Unreacted Glucose in Reaction Mix |
|---|---|
| 0 | 1.7 |
| 15 (before) | 0.7 |
| 15 (after) | 4.4 (calculated) |
| 45 (before) | 1.6 |
| 45 (after) | 5.0 |
| 60 (before) | 1.2 |
| 60 (after) | 4.6 (calculated) |
| 90 (end) | 1.5 |

Average unreacted glucose level 2.6

The neutralized product is analyzed by gas chromatography and is found to contain the following: 65.1% unreacted alcohol, 1.5% unreacted glucose, 1.0% polysaccharide, 20.1% alkyl monoglycoside, 7.0% alkyldiglycoside, 3.2% alkyl triglycoside and 1.9% tetraglycoside (results normalized to 100%). If this reaction product is distilled in a Pope Wiped Film Evaporator operated at 160-170°C and 2 mm pressure to reduce the unreacted alcohol content to 0.5%, a product will be obtained containing 4.3% unreacted glucose, 2.9% polysaccharide, 57.6% alkyl monoglycoside, 20.0% alkyl diglycoside, 9.2% alkyl triglycoside, 5.4% alkyl tetraglycoside and 0.5% unreacted alcohol.

- 1 -

0096917

CLAIMS

1.    A process for preparing alkyl glycosides, comprising reacting a reducing monosaccharide with at least a 50% molar excess of $C_8$ to $C_{25}$ monohydric alcohol in the presence of an acid catalyst at a temperature of from about 80°C to about 150°C and continuously removing the water of reaction from the system, wherein the monosaccharide is continuously or incrementally added to the alcohol and catalyst at a rate such that the amount of unreacted monosaccharide in the reaction mix is no more than about 10% by weight of said mix at any time and the average amount of unreacted monosaccharide in the said mix during the course of the reaction does not exceed about 5% by weight of said mix.

2.    The process of Claim 1 wherein the rate of addition of monosaccharide is such that the amount of unreacted monosaccharide in the reaction mix is no more than about 5% by weight of said mix at any time and the average amount of unreacted monosaccharide in the said mix during the course of the reaction does not exceed about 3% by weight of said mix.

3.    The process of Claim 2 wherein the alcohol contains from 12 to 18 carbon atoms and the molar ratio of alcohol to monosaccharide is from about 2.5:1 to about 5:1.

4.    The process of Claim 3 wherein the reaction temperature is from about 100°C to about 140°C.

5.    The process of Claim 4 wherein the water of reaction is removed by distillation and the reaction is conducted under a reduced pressure of from about 0.001 to about 50 mm of mercury.

6.      The process of Claim 5 wherein the monosaccharide is glucose and wherein the alcohol is a straight chain alcohol containing from about 12 to about 14 carbon atoms.

7.      The process of Claim 6 wherein the addition of monosaccharide is continuous.

8.      A process for preparing alkyl glycosides, comprising reacting a reducing monosaccharide with at least a 50% molar excess of $C_8$ to $C_{25}$ monohydric alcohol in the presence of an acid catalyst at a temperature of from about 80°C to about 150°C and continuously removing the water of reaction from the system, wherein the monosaccharide is continuously or incrementally added to the alcohol and catalyst at a rate which is sufficiently low to maintain the reaction system in a substantially single phase condition.

9.      The process of Claim 8 wherein the alcohol contains from 12 to 18 carbon atoms and the molar ratio of alcohol to monosaccharide is from about 2.5:1 to about 5:1.

10.      The process of Claim 9 wherein the reaction temperature is from about 100°C to about 140°C.

11.      The process of Claim 10 wherein the water of reaction is removed by distillation and the reaction is conducted under a reduced pressure of from about 0.001 to about 50 mm of mercury.

12.      The process of Claim 11 wherein the monosaccharide is glucose and wherein the alcohol is a straight chain alcohol containing from about 12 to about 14 carbon atoms.

13.     The process of Claim 12 wherein the addition of monosaccharide is continuous.

RLH/rmj

0096917

Application number

EP 83 20 0771

# European Patent Office

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A,D | US-A-3 839 318 (R.C. MANSFIELD) * Claims 1-3 * ----- | 1 | C 07 H 15/04 |
| | | | **TECHNICAL FIELDS SEARCHED (Int. Cl. ³)** |
| | | | C 07 H 15/00 |

The present search report has been drawn up for all claims

| Place of search | Date of completion of the search | Examiner |
|---|---|---|
| THE HAGUE | 14-09-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82